(19) Europäisches Patentamt
European Patent Office
Office européen des brevets

(11) **EP 2 962 621 A1**

(12) **EUROPEAN PATENT APPLICATION**
published in accordance with Art. 153(4) EPC

(43) Date of publication:
06.01.2016 Bulletin 2016/01

(51) Int Cl.:
*A61B 1/04* (2006.01)     *A61B 1/00* (2006.01)
*G02B 23/24* (2006.01)

(21) Application number: 13876108.5

(22) Date of filing: 24.09.2013

(86) International application number:
PCT/JP2013/075627

(87) International publication number:
WO 2014/132474 (04.09.2014 Gazette 2014/36)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(30) Priority: 26.02.2013 JP 2013035729

(71) Applicant: Olympus Corporation
Shibuya-ku
Tokyo 151-0072 (JP)

(72) Inventor: MITSUI, Masanori
Tokyo 151-0072 (JP)

(74) Representative: Gunzelmann, Rainer
Wuesthoff & Wuesthoff
Patentanwälte PartG mbB
Schweigerstraße 2
81541 München (DE)

(54) **IMAGE PROCESSING DEVICE, ENDOSCOPE DEVICE, IMAGE PROCESSING METHOD, AND IMAGE PROCESSING PROGRAM**

(57) An image processing device, an endoscope apparatus, an image processing method, an image processing program, and the like make it possible to perform an appropriate enhancement process corresponding to the observation state. The image processing device includes an image acquisition section (310) that acquires a captured image that includes an image of an object, a motion amount acquisition section (360) that acquires a motion amount of the object, a known characteristic information acquisition section (390) that acquires known characteristic information that is information that represents known characteristics relating to the object, and an enhancement processing section (370) that performs an enhancement process that corresponds to the motion amount based on the known characteristic information.

FIG. 1

EP 2 962 621 A1

**Description**

TECHNICAL FIELD

The present invention relates to an image processing device, an endoscope apparatus, an image processing method, an image processing program, and the like.

BACKGROUND ART

When observing tissue using an endoscope apparatus, and making a diagnosis, whether or not an early lesion has occurred is generally determined by observing tissue as to the presence or absence of minute concavities and convexities, or determining a difference in color (e.g., reddening or discoloration). When using an industrial endoscope apparatus instead of a medical endoscope apparatus, it is useful to observe the object (i.e., the surface of the object in a narrow sense) as to the presence or absence of a concave-convex structure or the like. In this case, it is possible to detect whether or not a crack has occurred in the inner side of a pipe that is difficult to directly observe with the naked eye, for example. It is also normally useful to detect the presence or absence of a concave-convex structure or the like of the object from the processing target image when using an image processing device other than an endoscope apparatus.

An endoscope apparatus may be designed to perform an image enhancement process that allows the user to easily observe the structure of the object and a difference in color. For example, Patent Document 1 discloses a method that enhances the structure of the object by image processing. Patent Document 2 discloses a method that utilizes a color enhancement process that allows the user to determine a lesion area.

RELATED-ART DOCUMENT

PATENT DOCUMENT

Patent Document 1: JP-A-2003-88498
Patent Document 2: JP-A-2005-342234

SUMMARY OF THE INVENTION

TECHNICAL PROBLEM

When an identical enhancement process is performed irrespective of the observation state, the resulting image may not be suitable for observation. For example, when using a medical endoscope apparatus, the image moving speed differs between screening observation and zoom observation, and the observation target of the doctor also differs between screening observation and zoom observation. Therefore, it is desirable to perform an appropriate enhancement process corresponding to the observation state.

Several aspects of the invention may provide an image processing device, an endoscope apparatus, an image processing method, an image processing program, and the like that make it possible to perform an appropriate enhancement process corresponding to the observation state.

SOLUTION TO PROBLEM

According to one aspect of the invention, there is provided an image processing device comprising:

an image acquisition section that acquires a captured image that includes an image of an object;
a motion amount acquisition section that acquires a motion amount of the object;
a known characteristic information acquisition section that acquires known characteristic information, the known characteristic information being information that represents known characteristics relating to the object; and
an enhancement processing section that performs an enhancement process that corresponds to the motion amount based on the known characteristic information.

According to one aspect of the invention, the enhancement process that corresponds to the motion amount of the object is performed based on the known characteristic information (i.e., information that represents known characteristics relating to the object). This makes it possible to perform an appropriate enhancement process corresponding to the observation state.

According to another aspect of the invention, there is provide an image processing method comprising:

acquiring a captured image that includes an image of an object;
acquiring a motion amount of the object;
acquiring known characteristic information, the known characteristic information being information that represents known characteristics relating to the object; and
performing an enhancement process that corresponds to the motion amount based on the known characteristic information.

According to another aspect of the invention, there is provided an image processing program that causes a computer to perform steps of:

acquiring a captured image that includes an image of an object;
acquiring a motion amount of the object;
acquiring known characteristic information, the known characteristic information being information that represents known characteristics relating to the object; and
performing an enhancement process that corresponds to the motion amount based on the known characteristic information.

BRIEF DESCRIPTION OF DRAWINGS

FIG. 1 illustrates a configuration example of an image processing device.
FIG. 2 illustrates a configuration example of an endoscope apparatus.
FIG. 3 illustrates a detailed configuration example of an image processing section (first embodiment).
FIG. 4 illustrates a detailed configuration example of a distance information acquisition section.
FIG. 5 illustrates a modified configuration example of an endoscope apparatus.
FIG. 6 illustrates a detailed configuration example of an enhancement processing section (first embodiment).
FIGS. 7A to 7D are views illustrating a process that extracts extracted concavity-convexity information using a filtering process.
FIGS. 8A to 8F are views illustrating a process that extracts extracted concavity-convexity information using a morphological process.
FIG. 9 illustrates an example of a flowchart of image processing (first embodiment).
FIG. 10 illustrates a detailed configuration example of an image processing section (second embodiment).
FIG. 11 illustrates a detailed configuration example of an enhancement processing section (second embodiment).
FIG. 12 illustrates an example of a flowchart of image processing (second embodiment).

DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

1. Outline

When examining the digestive tract using an imaging device (e.g., endoscope apparatus), and determining the presence or absence of an early lesion, weight is attached to the structure (e.g., concavities and convexities) of the surface of tissue, and a difference in color (e.g., reddening or discoloration). A method that enhances the color and contrast by spraying a dye onto the digestive tract may be used so that the doctor can more easily observe the structure of the object.

However, since the dye spraying operation is cumbersome for the doctor, and increases the burden imposed on the patient, attempts have been made that enhance the color or the concave-convex structure by image processing so that a lesion can be easily detected (determined) (see Patent Documents 1 and 2, for example).

When enhancing a color or a concave-convex structure by image processing, an image that is easy to observe (i.e., suitable for observation) can be obtained by changing the enhancement process corresponding to the observation state (observation method). For example, the doctor observes a relatively large structure when sequentially observing the entire digestive tract while relatively quickly moving the imaging device (scope) (e.g., during screening observation). Therefore, it is necessary to present the large structure within the image so that the large structure is not missed. On the other hand, the doctor observes a microscopic structure when the doctor has determined the target by screening, and closely observes the structure of the target by zoom observation (close observation). Therefore, it is necessary to enhance the microscopic structure so that the doctor can determine whether the target is benign or malignant.

However, it is troublesome to manually change (switch) the enhancement process corresponding to the observation state. If the enhancement process is not changed corresponding to the observation state, and a large structure and a microscopic structure are always enhanced, it is difficult to observe the enhanced microscopic structure during screening observation since the image moves quickly, and it is also difficult to observe the enhanced microscopic structure during zoom observation since the large structure is also enhanced.

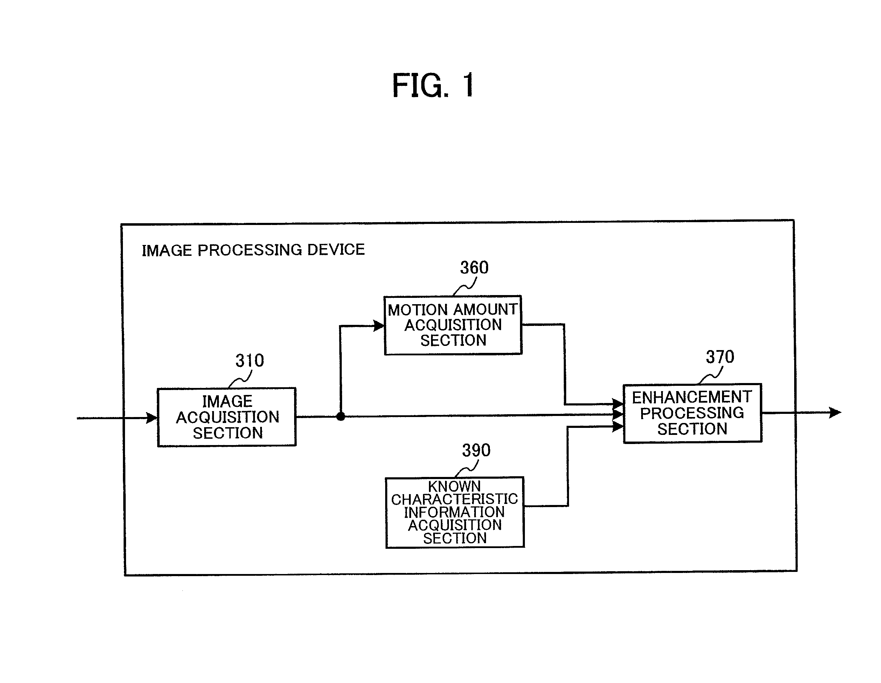

FIG. 1 illustrates a configuration example of an image processing device that can solve the above problem. The image processing device includes an image acquisition section 310 that acquires a captured image that includes an image of an object, a motion amount acquisition section 360 that acquires a motion amount of the object, a known characteristic information acquisition section 390 that acquires known characteristic information that is information that represents known characteristics relating to the object, and an enhancement processing section 370 that performs an enhancement process that corresponds to the motion amount based on the known characteristic information.

Since the motion amount of the object differs corresponding to the observation state (i.e., differs between different observation states), it is possible to change the enhancement process corresponding to the observation state by performing the enhancement process that corresponds to the motion amount. This makes it possible to selectively enhance the enhancement target in each observation state to provide an image that is suitable for observation.

The enhancement processing section 370 performs the enhancement process that corresponds to the motion amount on the object that agrees with the characteristics represented by the known characteristic information (described later).

More specifically, when the enhancement processing section 370 has determined that the motion amount falls under a motion amount that corresponds to screening observation, the enhancement processing section 370 performs a first enhancement process as the enhancement process on the object that agrees with the characteristics represented by the known characteristic information. When the enhancement processing section 370 has determined that the motion amount falls under a motion amount that corresponds to close observation, the enhancement processing section 370 performs a second enhancement process as the enhancement process on the object that agrees with the characteristics represented by the known characteristic information, the second enhancement process differing from the first enhancement process.

This makes it possible to perform the enhancement process that corresponds to the motion amount when performing the enhancement process on the target that agrees with the characteristics represented by the known characteristic information so that the target is easily observed in each observation state. The target may be a concave-convex part, a reddened part, or a discolored part of tissue (described later). The user pays attention to a specific feature (e.g., a large structure, a small structure, or an area in a given color) in each observation state. The enhancement process may enhance such a feature to which the user pays attention corresponding to the observation state. For example, a relatively large concave-convex structure is enhanced during screening observation in which the motion amount of the object is large, and a minute (microscopic) concave-convex structure is enhanced during zoom observation in which the motion amount of the object is small.

Note that the term "motion amount" used herein refers to the motion amount of the object within the captured image (e.g., the motion direction or the motion distance of the object within the captured image). The motion direction and the motion distance may be acquired as the motion vector, or only the motion distance may be acquired as the motion amount when it suffices to determine the motion velocity. The motion amount is obtained by performing a matching process on the feature point of the captured image between two frames, for example.

The term "known characteristic information" used herein refers to information by which a useful structure of the surface of the object can be distinguished from an unuseful structure of the surface of the object. Specifically, the known characteristic information may be information (e.g., the size of a concave-convex part specific to a lesion, hue, or chroma) about a concave-convex part or a color for which the enhancement process is useful (e.g., a concave-convex part or a color that is useful for finding an early lesion). In this case, an object that agrees with the known characteristic information is determined to be the enhancement target. Alternatively, the known characteristic information may be information about a structure for which the enhancement process is not useful. In this case, an object that does not agree with the known characteristic information is determined to be the enhancement target. Alternatively, information about a useful concave-convex part and information about an unuseful structure may be stored, and the range of the useful concave-convex part may be set with high accuracy.

2. First embodiment

2.1. Endoscope apparatus

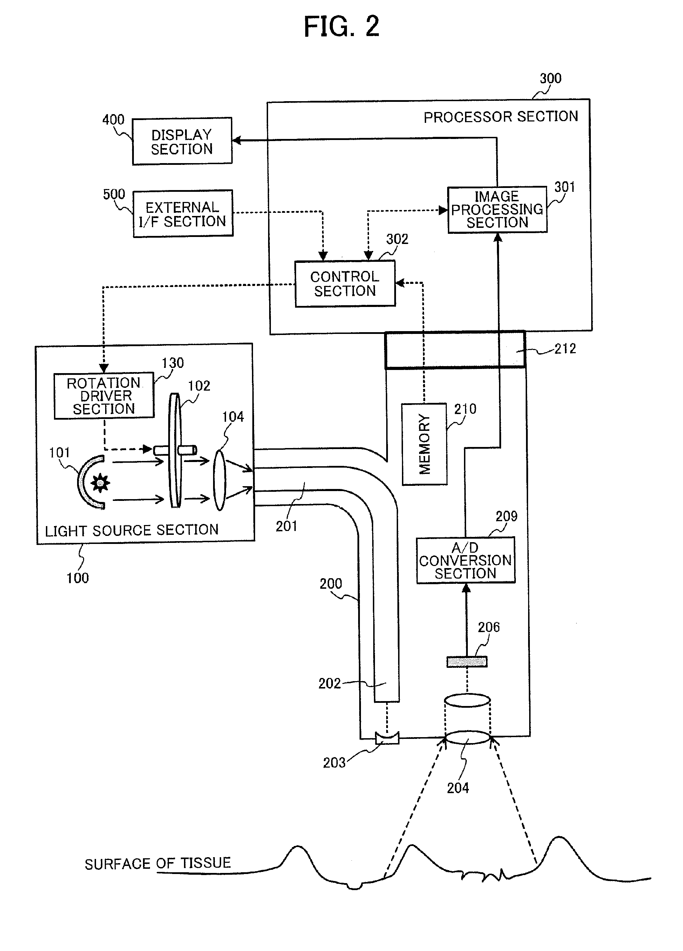

A first embodiment of the invention is described in detail below. FIG. 2 illustrates a configuration example of an endoscope apparatus according to the first embodiment. The endoscope apparatus includes a light source section 100, an imaging section 200, a processor section 300 (control device), a display section 400, and an external I/F section 500.

The light source section 100 includes a white light source 101, a rotary color filter 102 that includes a plurality of color filters that differ in spectral transmittance, a rotation driver section 103 that drives the rotary color filter 102, and a condenser lens 104 that focuses light (that has passed through the rotary color filter 102 and differs in spectral characteristics) on the incident end face of a light guide fiber 201. The rotary color filter 102 includes three primary-color filters (red color filter, green color filter, and blue color filter), and a rotary motor.

The rotation driver section 103 rotates the rotary color filter 102 at a given rotational speed in synchronization with the imaging period of an image sensor 206 included in the imaging section 200 based on a control signal output from a control section 302 included in the processor section 300. For example, when the rotary color filter 102 is rotated at 20 revolutions per second, each color filter crosses incident white light every 1/60th of a second. In this case, the image sensor 206 captures reflected light from the observation target to which red (R), green (G), or blue (B) light has been applied, and transmits the captured image to an A/D conversion section 209 every 1/60th of a second. Specifically, the endoscope apparatus according to the first embodiment frame-sequentially captures an R image, a G image, and a B image every 1/60th of a second, and the substantial frame rate is 20 fps.

Note that the first embodiment is not limited to the frame sequential method. For example, white light emitted from the white light source 101 may be applied to the object, and captured using an image sensor that includes an RGB Bayer color filter array.

The imaging section 200 is formed to be elongated and flexible so that the imaging section 200 can be inserted into a body cavity (e.g., stomach or large intestine), for example. The imaging section 200 includes the light guide fiber 201 that guides the light focused by the light source section 100, and an illumination lens 203 that diffuses the light guided by the light guide fiber 201 to illuminate the observation target. The imaging section 200 also includes an objective lens 204 that focuses the reflected light from the observation target, the image sensor 206 that detects the focused light, and an A/D conversion section 209 that converts photoelectrically converted analog signals output from the image sensor 206 into digital signals. The imaging section 200 further includes a memory 210 that stores scope ID information and specific information (including production variations) about the imaging section 200, and a connector 212 for removably connecting the imaging section 200 and the processor section 300.

The image sensor 206 is a single-chip monochrome image sensor when implementing the frame sequential method. For example, a CCD image sensor, a CMOS image sensor, or the like may be used as the image sensor 206. The A/D conversion section 209 converts the analog signals output from the image sensor 206 into digital signals, and outputs the digital signals (image) to the image processing section 301. The memory 210 is connected to the control section 302, and transmits the scope ID information and the specific information (including production variations) to the control section 302.

The processor section 300 includes an image processing section 301 that performs image processing on the image transmitted from the A/D conversion section 209, and the control section 302 that controls each section of the endoscope apparatus.

The display section 400 is a display device that can display a movie (moving image), and is implemented by a CRT, a liquid crystal monitor, or the like.

The external I/F section 500 is an interface that allows the user to input information or the like to the endoscope apparatus. For example, the external I/F section 500 includes a power switch (power ON/OFF switch), a shutter button (capture start button), a mode (e.g., imaging mode) switch (e.g., a switch for selectively performing an enhancement process on a concave-convex part of the surface of tissue), and the like. The external I/F section 500 outputs the input information to the control section 302.

2.2. Image processing section

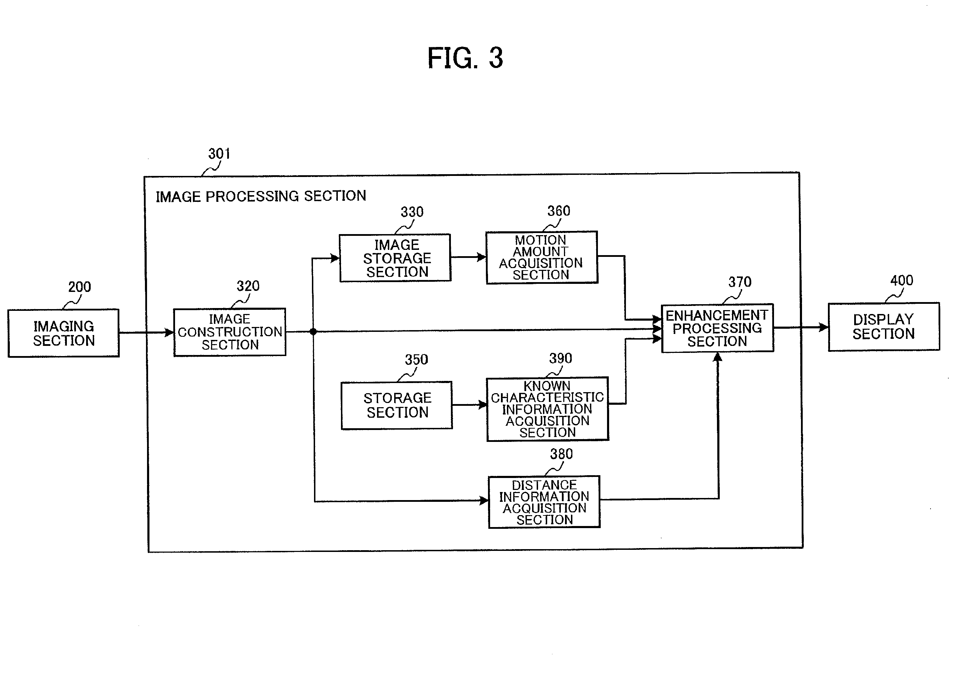

FIG. 3 illustrates a detailed configuration example of the image processing section 301. The image processing section 301 includes an image construction section 320, an image storage section 330, a storage section 350, a motion amount acquisition section 360, an enhancement processing section 370, a distance information acquisition section 380, and a known characteristic information acquisition section 390. Note that the image construction section 320 corresponds to the image acquisition section 310 illustrated in FIG. 1.

The image construction section 320 performs given image processing (e.g., OB process, gain process, and gamma process) on the image captured by the imaging section 200 to generate an image that can be output from the display section 400. The image construction section 320 outputs the generated image to the image storage section 330, the enhancement processing section 370, and the distance information acquisition section 380.

The image storage section 330 stores the image output from the image construction section 320 corresponding to a plurality of frames (i.e., a plurality of time-series (consecutive) frames).

The motion amount acquisition section 360 calculates the motion amount of the object within the captured image based on the images (corresponding to a plurality of frames) stored in the image storage section 330, and outputs the motion amount to the enhancement processing section 370. For example, the motion amount acquisition section 360 performs a matching process on the image corresponding to a reference frame and the image corresponding to the subsequent frame to calculate the motion vector between the images (frame images). The motion amount acquisition section 360 sequentially calculates a plurality of motion vectors over a plurality of frames while shifting the reference image, and calculates the average value of the plurality of motion vectors as the motion amount.

The known characteristic information acquisition section 390 reads (acquires) known characteristic information stored in the storage section 350, and outputs the known characteristic information to the enhancement processing section 370. The known characteristic information is information about the size (i.e., dimensional information (e.g., width, height, and depth)) of the concave-convex part of tissue to be determined to be the enhancement target.

The distance information acquisition section 380 acquires distance information about the distance to the object based on the captured image, and outputs the distance information to the enhancement processing section 370. The term "distance information" used herein refers to information in which each position within the captured image is linked to the distance to the object at each position within the captured image. For example, the distance information is a distance map. The term "distance map" used herein refers to a map in which the distance (depth) to the object in the Z-axis direction (i.e., the direction of the optical axis of the imaging section 200) is specified corresponding to each point (e.g., each pixel) in the XY plane, for example. The details of the distance information acquisition section 380 are described later.

The enhancement processing section 370 determines the target based on the known characteristic information and the distance information, performs the enhancement process that corresponds to the motion amount on the target, and outputs the resulting image to the display section 400. Specifically, the enhancement processing section 370 extracts a concave-convex part that agrees with the desired dimensional characteristics represented by the known characteristic information from the distance information, and determines the extracted concave-convex part to be the target. When the motion amount is larger than a threshold value (e.g., during screening observation), the enhancement processing section 370 enhances the extracted concave-convex part that has a size (concavity-convexity pattern) larger than a first size threshold value. When the motion amount is smaller than the threshold value (e.g., during zoom observation), the enhancement processing section 370 enhances the extracted concave-convex part that has a size (concavity-convexity pattern) smaller than a second size threshold value. The first size threshold value and the second size threshold value may be set taking account of the size of the enhancement target concave-convex part (that allows the user to easily observe the resulting image) in each observation state. The enhancement process enhances a different color component depending on whether the target is a concave part or a convex part, for example.

Note that the enhancement process need not necessarily be performed on the determined target. The enhancement process may be performed based on the known characteristic information without determining the target. For example, the filter characteristics (e.g., the enhancement target frequency band) of a structural enhancement process (e.g., an enhancement process that enhances a high-frequency component of an image) may be set based on the size of the concave-convex part represented by the known characteristic information so that a concave-convex part having the desired size is enhanced. In this case, the filter characteristics may be changed corresponding to the motion amount.

2.3. Distance information acquisition process

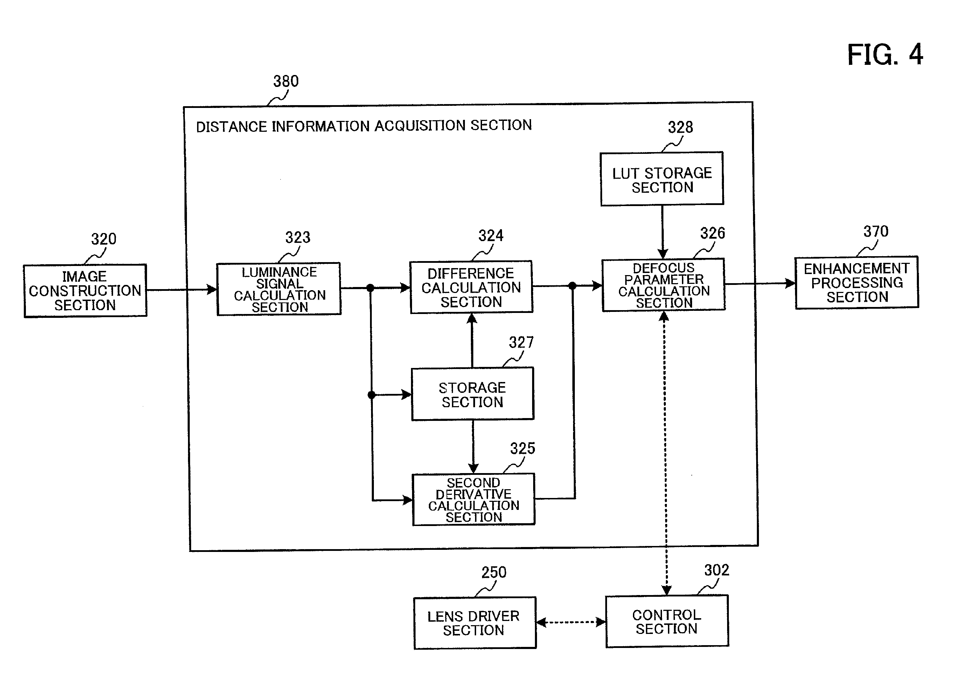

FIG. 4 illustrates a detailed configuration example of the distance information acquisition section 380. The distance information acquisition section 380 includes a luminance signal calculation section 323, a difference calculation section 324, a second derivative calculation section 325, a defocus parameter calculation section 326, a storage section 327, and an LUT storage section 328.

The luminance signal calculation section 323 calculates a luminance signal Y (luminance value) from the captured image output from the image acquisition section 310 using the following expression (1) under control of the control section 302.

$$Y = 0.299 \times R + 0.587 \times G + 0.114 \times B \qquad (1)$$

The calculated luminance signal Y is transmitted to the difference calculation section 324, the second derivative calculation section 325, and the storage section 327. The difference calculation section 324 calculates the difference between the luminance signals Y from a plurality of images necessary for calculating a defocus parameter. The second derivative calculation section 325 calculates the second derivative of the luminance signals Y of the image, and calculates the average value of the second derivatives obtained from a plurality of luminance signals Y that differ in the degree of defocus. The defocus parameter calculation section 326 calculates the defocus parameter by dividing the difference between the luminance signals Y of the image calculated by the difference calculation section 324 by the average value of the second derivatives calculated by the second derivative calculation section 325.

The storage section 327 stores the luminance signals Y of the first captured image, and the second derivative results thereof. Therefore, the distance information acquisition section 380 can place the focus lens at different positions through the control section 302, and acquire a plurality of luminance signals Y at different times. The LUT storage section 328 stores the relationship between the defocus parameter and the object distance in the form of a look-up table (LUT).

The object distance calculation method is described below. The control section 302 calculates the optimum focus lens position using a known contrast detection method, a known phase detection method, or the like based on the imaging mode set in advance using the external I/F section 500. The lens driver section 250 drives the focus lens to the calculated focus lens position based on a signal output from the control section 302. The image sensor 206 acquires the first image of the object at the focus lens position to which the focus lens has been driven. The acquired image is stored in the storage section 327 through the image acquisition section 310 and the luminance signal calculation section 323.

The lens driver section 250 then drives the focus lens to a second focus lens position that differs from the focus lens position at which the first image has been acquired, and the image sensor 206 acquires the second image of the object at the focus lens position to which the focus lens has been driven. The second image thus acquired is output to the distance information acquisition section 380 through the image acquisition section 310.

When the second image has been acquired, the defocus parameter is calculated. The difference calculation section 324 included in the distance information acquisition section 380 reads the luminance signals Y of the first image from the storage section 327, and calculates the difference between the luminance signal Y of the first image and the luminance signal Y of the second image output from the luminance signal calculation section 323.

The second derivative calculation section 325 calculates the second derivative of the luminance signals Y of the second image output from the luminance signal calculation section 323. The second derivative calculation section 325 then reads the luminance signals Y of the first image from the storage section 327, and calculates the second derivative of the luminance signals Y. The second derivative calculation section 325 then calculates the average value of the second derivative of the first image and the second derivative of the second image.

The defocus parameter calculation section 326 calculates the defocus parameter by dividing the difference calculated by the difference calculation section 324 by the average value of the second derivatives calculated by the second derivative calculation section 325.

The relationship between the defocus parameter and the focus lens position is stored in the LUT storage section 328 in the form of a table. The defocus parameter calculation section 326 calculates the object distance with respect to the optical system from the defocus parameter by linear interpolation using the defocus parameter and the information included in the table stored in the LUT storage section 328. The calculated object distance is output to the enhancement processing section 370 as the distance information.

Note that the distance information acquisition process may be implemented in various other ways. For example, the distance information may be acquired (calculated) by a Time-of-Flight method that utilizes infrared light or the like. When using the Time-of-Flight method, blue light may be used instead of infrared light, for example.

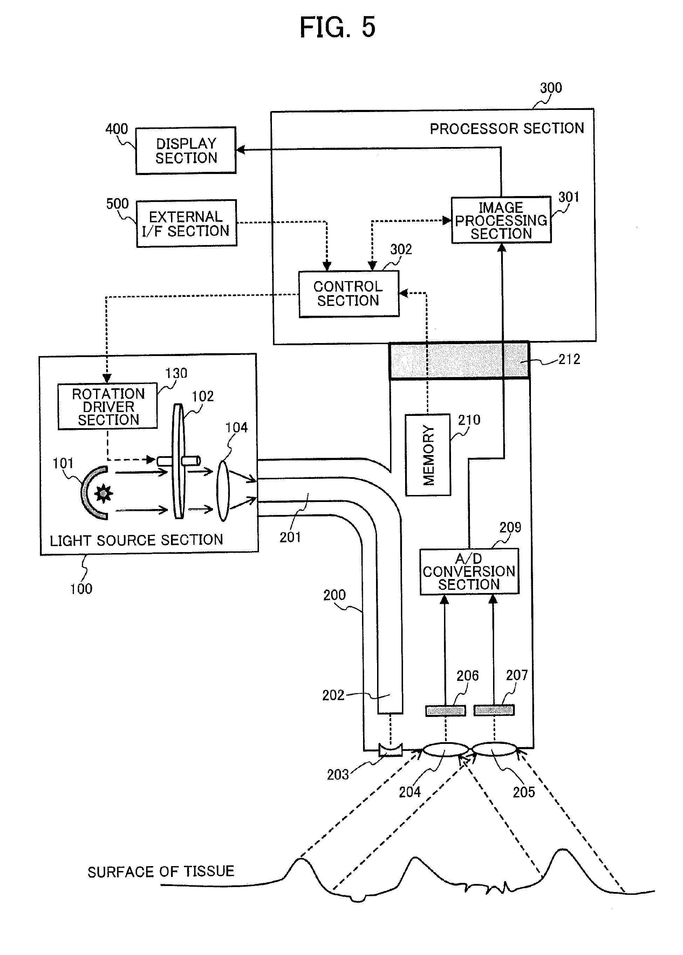

The distance information may be acquired using a stereo matching process. In this case, the endoscope apparatus may have the configuration illustrated in FIG. 5. As illustrated in FIG. 5, the imaging section 200 includes an objective lens 205 and an image sensor 207 in addition to the elements illustrated in FIG. 2.

The objective lenses 204 and 205 are disposed at given intervals so that a parallax image (hereinafter referred to as "stereo image") can be captured. The objective lenses 204 and 205 respectively form a left image and a right image (stereo image) on the image sensors 206 and 207. The A/D conversion section 209 performs the A/D conversion process on the left image and the right image respectively output from the image sensors 206 and 207, and outputs the resulting left image and the resulting right image to the image construction section 320 and the distance information acquisition section 380.

The distance information acquisition section 380 performs a matching calculation process on the left image (reference image) and the right image using an epipolar line that passes through an attention pixel of the reference image with respect to a local area that includes the attention pixel and a local area of the right image. The distance information acquisition section 380 calculates a position at which the correlation obtained by the matching calculation process becomes a maximum as a parallax, converts the parallax into the distance in the depth direction (i.e., the Z-axis direction of the distance map), and outputs the distance information to the enhancement processing section 370.

2.4. Target determination process and enhancement process

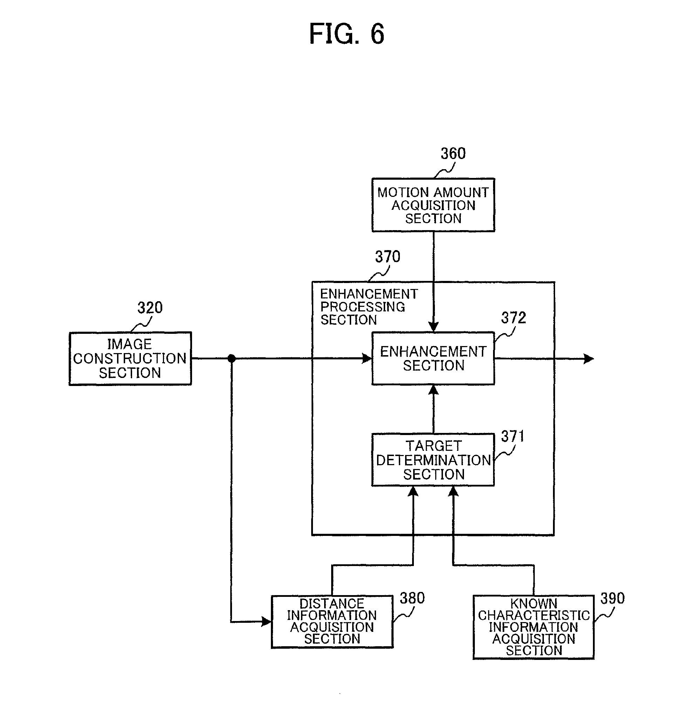

FIG. 6 illustrates a detailed configuration example of the enhancement processing section 370. The enhancement processing section 370 includes a target determination section 371 and an enhancement section 372. An example in which the target is determined based on the distance information and the known characteristic information, and the enhancement process is performed on the determined target, is described below. Note that the configuration is not limited thereto (see above).

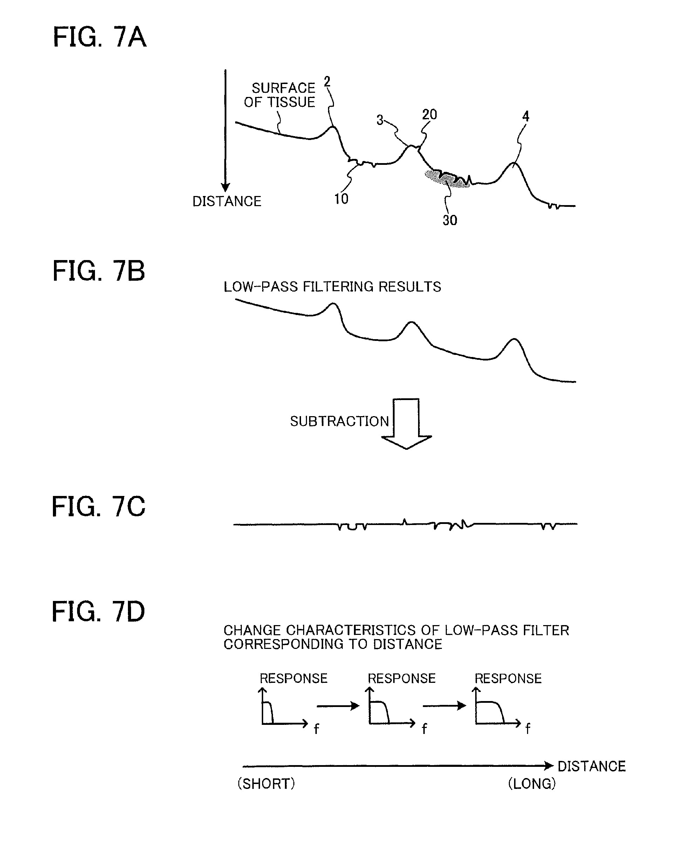

FIG. 7A schematically illustrates an example of the distance map. FIG. 7A illustrates an example in which the distance map is a one-dimensional distance map for convenience of explanation. In FIG. 7A, the arrow indicates the distance axis. The distance map includes information about an approximate structure of tissue (e.g., shape information about a lumen and folds 2, 3, and 4), and information about the concave-convex part (e.g., concave parts 10 and 30 and a convex part 20) in the surface area of tissue.

The known characteristic information acquisition section 390 acquires the dimensional information (i.e., information about the size of the extraction target concave-convex part of tissue) from the storage section 350 as the known characteristic information, and determines the frequency characteristics of a low-pass filtering process based on the dimensional information. As illustrated in FIG. 7B, the target determination section 371 performs the low-pass filtering process on the distance map using the determined frequency characteristics to extract the information about an approximate structure of tissue (shape information about a lumen, folds, and the like).

As illustrated in FIG. 7C, the target determination section 371 subtracts the information about an approximate structure of tissue from the distance map to generate a concavity-convexity map that is concavity-convexity information about the surface area of tissue (i.e., information about a concave-convex part having the desired size). For example, the horizontal direction of the image, the distance map, and the concavity-convexity map is defined as the x-axis, and the vertical direction of the image, the distance map, and the concavity-convexity map is defined as the y-axis. The distance at the coordinates (x, y) of the distance map is defined as dist(x, y), and the distance at the coordinates (x, y) of the distance map subjected to the low-pass filtering process is defined as dist_LPF(x, y). In this case, the concavity-convexity information diff(x, y) at the coordinates (x, y) of the concavity-convexity map is calculated by the following expression (2).

$$diff(x, y) = dist(x, y) - dist\_LPF(x, y) \qquad (2)$$

The enhancement processing section 370 outputs the concavity-convexity map calculated as described above to the enhancement processing section 370. In this example, a concave-convex part in the surface area of tissue is determined to be the enhancement target by extracting a concave-convex part in the surface area of tissue as the concavity-convexity map.

A process that determines the cut-off frequency (extraction process parameter in a broad sense) from the dimensional information is described in detail below.

The target determination section 371 performs the low-pass filtering process using a given size (e.g., $N \times N$ pixels (N is a natural number equal to or larger than 2)) on the input distance information. The target determination section 371 adaptively determines the extraction process parameter based on the resulting distance information (local average distance). Specifically, the target determination section 371 determines the characteristics of the low-pass filtering process that smooth the extraction target concave-convex part of tissue due to a lesion while maintaining the structure of the lumen and the folds specific to the observation target part. Since the characteristics of the extraction target (i.e., concave-convex part) and the exclusion target (i.e., folds and lumen) can be determined from the known characteristic information, the spatial frequency characteristics are known, and the characteristics of the low-pass filter can be determined. Since the apparent size of the structure changes corresponding to the local average distance, the characteristics of the low-pass filter are determined corresponding to the local average distance (see FIG. 7D).

The low-pass filtering process is implemented by a Gaussian filter represented by the following expression (3), or a bilateral filter represented by the following expression (4), for example. The frequency characteristics of these filters are controlled using $\sigma$, $\sigma_c$, and $\sigma_v$. A $\sigma$ map that corresponds to the pixels of the distance map on a one-to-one basis may be generated as the extraction process parameter. When using the bilateral filter, either or both of a $\sigma_c$ map and a $\sigma_v$ map may be generated as the extraction process parameter.

$$f(x) = \frac{1}{N} \exp\left( -\frac{(x - x0)^2}{2\sigma^2} \right) \qquad (3)$$

$$f(x) = \frac{1}{N} \exp\left( -\frac{(x-x0)^2}{2\sigma_c^2} \right) \times \exp\left( -\frac{(p(x)-p(x0))^2}{2\sigma_v^2} \right) \qquad (4)$$

For example, $\sigma$ may be a value that is larger than a value obtained by multiplying a pixel-to-pixel distance D1 of the distance map corresponding to the size of the extraction target concave-convex part by $\alpha$ (>1), and is smaller than a value obtained by multiplying a pixel-to-pixel distance D2 of the distance map corresponding to the size of the lumen and the folds specific to the observation target part by $\beta$ (<1). For example, $\sigma$ may be calculated by $\sigma=(\alpha*D1+\beta*D2)/2*R\sigma$. Note that $R\sigma$ is a function of the local average distance. The value $R\sigma$ increases as the local average distance decreases, and decreases as the local average distance increases.

The known characteristic information acquisition section 390 may read the dimensional information corresponding to the observation target part from the storage section 350, and the target determination section 371 may determine the target corresponding to the observation target part based on the dimensional information, for example. The observation target part may be determined using the scope ID stored in the memory 210 (see FIG. 2), for example. For example, when the scope is an upper gastrointestinal scope, the dimensional information corresponding to the gullet, the stomach, and the duodenum (i.e., observation target part) is read from the storage section 350. When the scope is a lower gastrointestinal scope, the dimensional information corresponding to the large intestine (i.e., observation target part) is read from the storage section 350.

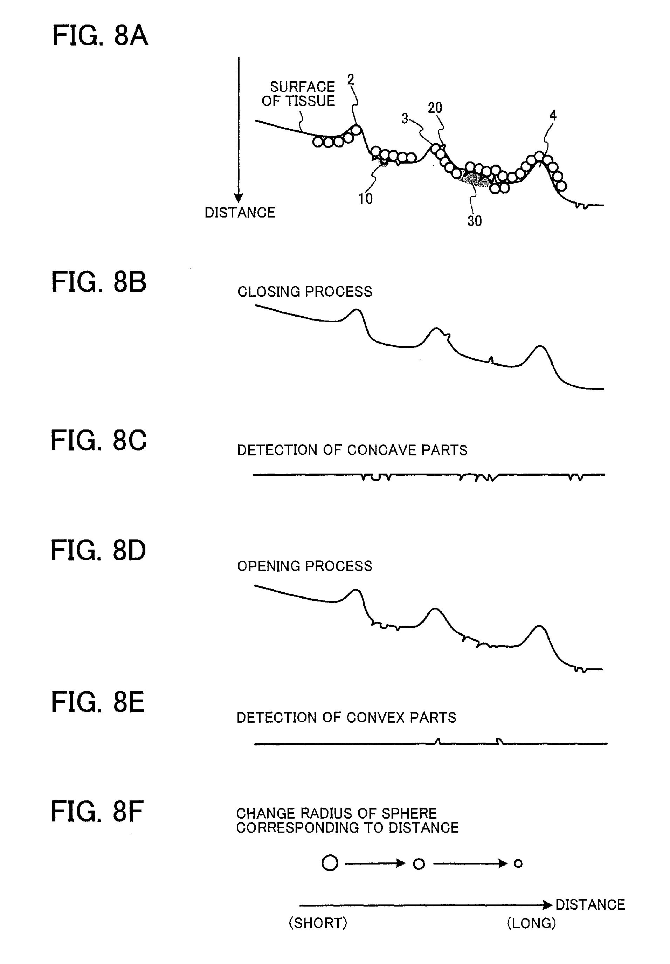

Note that the extraction process is not limited to the extraction process that utilizes the low-pass filtering process. For example, extracted concavity-convexity information may be acquired using a morphological process. In this case, an opening process and a closing process using a given kernel size (i.e., the size (sphere diameter) of a structural element) are performed on the distance map (see FIG. 8A). The extraction process parameter is the size of the structural element. For example, when using a sphere as the structural element, the diameter of the sphere is set to be smaller than the size of the lumen and the folds specific to the observation target part based on observation target part information, and larger than the size of the extraction target concave-convex part of tissue due to a lesion. As illustrated in FIG. 8F, the diameter of the sphere is increased as the local average distance decreases, and is decreased as the local average distance increases. As illustrated in FIGS. 8B and 8C, the concave parts of the surface of tissue are extracted by calculating the difference between information obtained by the closing process and the original distance information. As illustrated in FIGS. 8D and 8E, the convex parts of the surface of tissue are extracted by calculating the difference between information obtained by the opening process and the original distance information.

The enhancement section 372 determines the observation state (observation method) based on the motion amount, and performs the enhancement process that corresponds to the observation state. Specifically, when the enhancement section 372 has determined that the motion amount is large, and screening observation is being performed, the enhancement section 372 enhances the extracted concave-convex part that has a large size. When the enhancement section 372 has determined that the motion amount is small, and zoom observation is being performed, the enhancement section 372 enhances the extracted concave-convex part that has a small size.

A process that enhances a different color depending on whether the target is a concave part or a convex part is described below as an example of the enhancement process. A pixel for which the concavity-convexity information diff(x, y) is smaller than 0 (diff(x, y)<0) is determined to be a convex part, and a pixel for which the concavity-convexity information diff(x, y) is larger than 0 (diff(x, y)>0) is determined to be a concave part (see the expression (2)). A chroma enhancement process that corresponds to a given hue is performed on each pixel that has been determined to be a convex part, and a chroma enhancement process that corresponds to a hue that differs from the given hue is performed on each pixel that has been determined to be a concave part, for example. The size of a concave-convex part may be determined by comparing the width (i.e., the number of pixels) of a convex area and the width (i.e., the number of pixels) of a concave area with a threshold value, for example. Note that the enhancement process is not limited thereto. Various other enhancement processes may also be used. For example, a given color (e.g., blue) may be enhanced to a larger extent as the concavity-convexity information diff(x, y) increases (i.e., the depth increases) to simulate a state in which a dye (e.g., indigo carmine) has been sprayed. The coloring method may be changed depending on whether the observation state is a screening observation state or a zoom observation state.

According to the first embodiment, the target determination section 371 determines the extraction process parameter based on the known characteristic information, and determines a concave-convex part of the object to be the enhancement target based on the determined extraction process parameter.

This makes it possible to perform the concavity-convexity information extraction (separation) process (target determination process) using the extraction process parameter determined based on the known characteristic information. In order to accurately extract the extracted concavity-convexity information, it is necessary to perform a control process that extracts information about the desired concave-convex part from the information about various structures included in the distance information while excluding other structures (e.g., the structures (e.g., folds) specific to tissue).

In the first embodiment, such a control process is implemented by setting the extraction process parameter based on the known characteristic information.

The target determination section 371 may determine the size of the structural element used for the opening process and the closing process as the extraction process parameter based on the known characteristic information, and perform the opening process and the closing process using the structural element having the determined size to extract a concave-convex part of the object as the extracted concavity-convexity information.

This makes it possible to extract the extracted concavity-convexity information based on the opening process and the closing process (morphological process in a broad sense). In this case, the extraction process parameter is the size of the structural element used for the opening process and the closing process. When the structural element is a sphere, the extraction process parameter is a parameter that represents the diameter of the sphere, for example.

The captured image may be an *in vivo* image that is obtained by capturing the inside of a living body, the object may include a global three-dimensional structure that is a lumen structure inside the living body, and a local concave-convex structure that is formed on the lumen structure, and is more local than the global three-dimensional structure, and the target determination section 371 may extract the concave-convex part of the object that is selected from the global three-dimensional structure and the local concave-convex structure included in the object, and agrees with the characteristics represented by the known characteristic information, as the extracted concavity-convexity information.

This makes it possible to implement a process that extracts the global three-dimensional structure (i.e., a structure having a low spatial frequency as compared with the concave-convex part) and the concave-convex part (that is smaller than the global three-dimensional structure) included in the distance information when applying the method according to the first embodiment to an *in vivo* image. For example, when the extraction target is a concave-convex part that is useful for finding an early lesion, the three-dimensional structure (e.g., folds and a structure based on the curvature of a wall surface) of tissue can be excluded from the extraction target, and the target determination section 371 extracts only the extraction target concave-convex part. In this case, since the global structure (i.e., a structure having a low spatial frequency) is excluded from the extraction target, and the local structure (i.e., a structure having a high spatial frequency) is determined to be the extraction target, an intermediate spatial frequency or the like is set to be the boundary, for example.

2.5. Software

Although an example in which each section included in the processor section 300 is implemented by hardware has been described above, the configuration is not limited thereto. For example, a CPU may perform the process of each section on image signals (acquired in advance using an imaging device) and the distance information. Specifically, the process of each section may be implemented by software by causing the CPU to execute a program. Alternatively, part of the process of each section may be implemented by software.

In this case, a program stored in an information storage medium is read, and executed by a processor (e.g., CPU). The information storage medium (computer-readable medium) stores a program, data, and the like. The function of the information storage medium may be implemented by an optical disk (e.g., DVD or CD), a hard disk drive (HDD), a memory (e.g., memory card or ROM), or the like. The processor (e.g., CPU) performs various processes according to the first embodiment based on the program (data) stored in the information storage medium. Specifically, a program that causes a computer (i.e., a device that includes an operation section, a processing section, a storage section, and an output section) to function as each section according to the first embodiment (i.e., a program that causes a computer to execute the process of each section according to the first embodiment) is stored in the information storage medium.

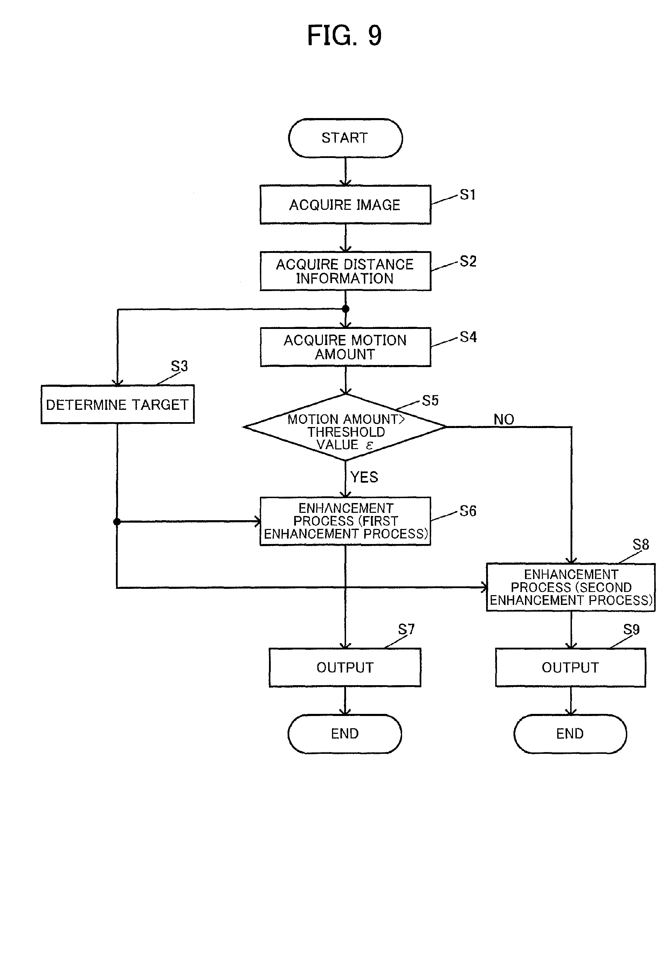

FIG. 9 is a flowchart when the process performed by the image processing section 301 is implemented by software. The captured image is acquired (step S1), and the distance information when the captured image was captured is acquired (step S2).

The target is determined using the above method (step S3). The motion amount of the object is calculated from the captured image (step S4), and whether or not the motion amount (e.g., the magnitude of the motion vector) is larger than the threshold value $\varepsilon$ is determined (step S5). When the motion amount is larger than the threshold value $\varepsilon$ (i.e., when it has been determined that screening observation is being performed), a first enhancement process is performed on the captured image (step S6), and the resulting image is output (step S7). The first enhancement process enhances the determined target that has a size larger than a first size threshold value Tk. When the motion amount is equal to or smaller than the threshold value $\varepsilon$ (i.e., when it has been determined that zoom observation is being performed), a second enhancement process is performed on the captured image (step S8), and the resulting image is output (step S9). The second enhancement process enhances the determined target that has a size smaller than a second size threshold value Ts.

According to the first embodiment, the distance information acquisition section 380 acquires the distance information (distance map in a narrow sense) that represents the distance from the imaging section 200 to the object when the imaging section 200 captured the captured image. The enhancement processing section 370 performs the enhancement process that corresponds to the motion amount based on the distance information and the known characteristic information.

This makes it possible to perform the enhancement process based on the distance information and the known characteristic information, and change the enhancement process corresponding to the observation state. It is possible to determine the observation target that has a specific three-dimensional structure or shape by utilizing the distance information, for example.

The known characteristic information acquisition section 390 may acquire the known characteristic information that is information (e.g., the size of a structure specific to a lesion) that represents the known characteristics relating to the structure of the object. The enhancement processing section 370 may perform the enhancement process on a concave-convex part of the object that agrees with the characteristics represented by the known characteristic information.

This makes it possible to change the enhancement process corresponding to the motion amount so that the concave-convex part to which the user pays attention in each observation state is enhanced. For example, the target determination section 371 extracts a concave-convex part that has the desired characteristics (e.g., size) from the distance information, and determines the extracted concave-convex part to be the enhancement target. The enhancement processing section 370 performs the enhancement process on the determined target. This makes it possible to change the characteristics (e.g., size) of the enhancement target concave-convex part corresponding to the motion amount. Note that the target determination section 371 need not necessarily be provided. The enhancement process may be performed based on the known characteristic information without determining the target.

More specifically, when the motion amount (e.g., the magnitude of the motion vector) is larger than the threshold value $\varepsilon$, the enhancement processing section 370 performs the enhancement process on the extracted concave-convex part that has been determined to have a size larger than the first size threshold value Tk. When the motion amount is smaller than the threshold value $\varepsilon$, the enhancement processing section 370 performs the enhancement process on the extracted concave-convex part that has been determined to have a size smaller than the second size threshold value Ts.

According to this configuration, since a small structure is not enhanced during screening observation in which the object (image) moves quickly, it is possible to reduce complexity during observation. Since a small structure is enhanced during zoom observation in which the object (image) moves slowly, it is possible for the user to closely observe a minute lesion structure. It is thus possible to improve visibility in each observation state, and suppress a situation in which a lesion is missed while improving the diagnostic accuracy.

3. Second embodiment

The second embodiment illustrates an example in which a reddened part or a discolored part is enhanced. Since a reddened part and a discolored part may not have a specific shape, differing from a concave-convex part, it is desirable to enhance a reddened part and a discolored part using a method that differs from the method employed when enhancing a concave-convex part. Note that the term "reddened part" used herein refers to a part that is observed to have a high degree of redness as compared with its peripheral area, and the term "discolored part" used herein refers to a part that is observed to have a low degree of redness as compared with its peripheral area.

A detailed configuration according to the second embodiment is described below. An endoscope apparatus according to the second embodiment may be configured in the same manner as the endoscope apparatus according to the first embodiment. Note that the same elements as those described above in connection with the first embodiment are respectively indicated by the same reference signs (symbols), and description thereof is appropriately omitted.

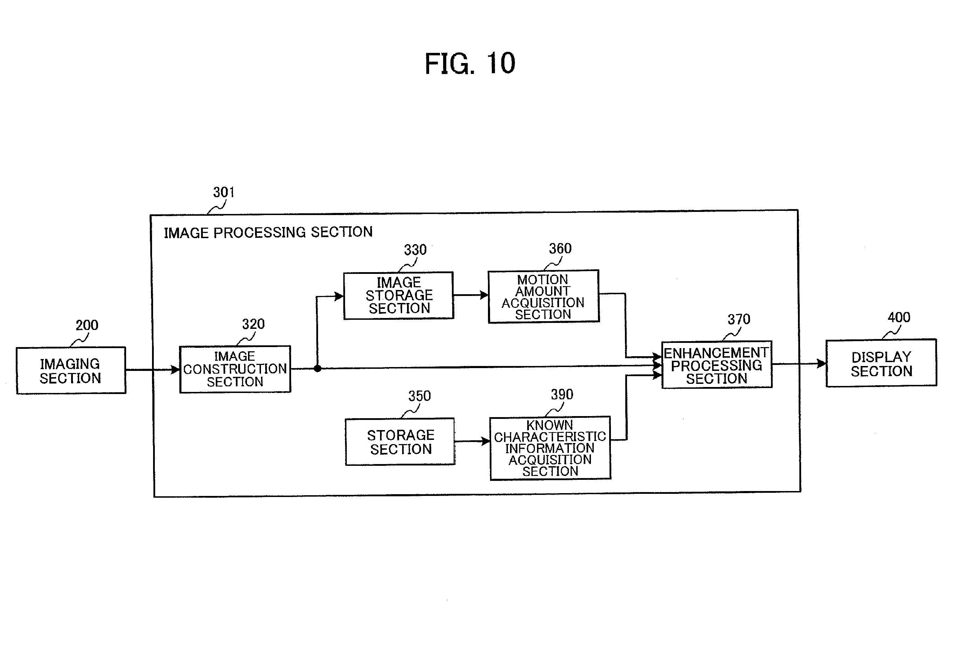

FIG. 10 illustrates a detailed configuration example of the image processing section 301 according to the second embodiment. The image processing section 301 includes an image construction section 320, an image storage section 330, a storage section 350, a motion amount acquisition section 360, an enhancement processing section 370, and a known characteristic information acquisition section 390. The image processing section 301 according to the second embodiment differs from the image processing section 301 according to the first embodiment in that the distance information acquisition section 380 is omitted.

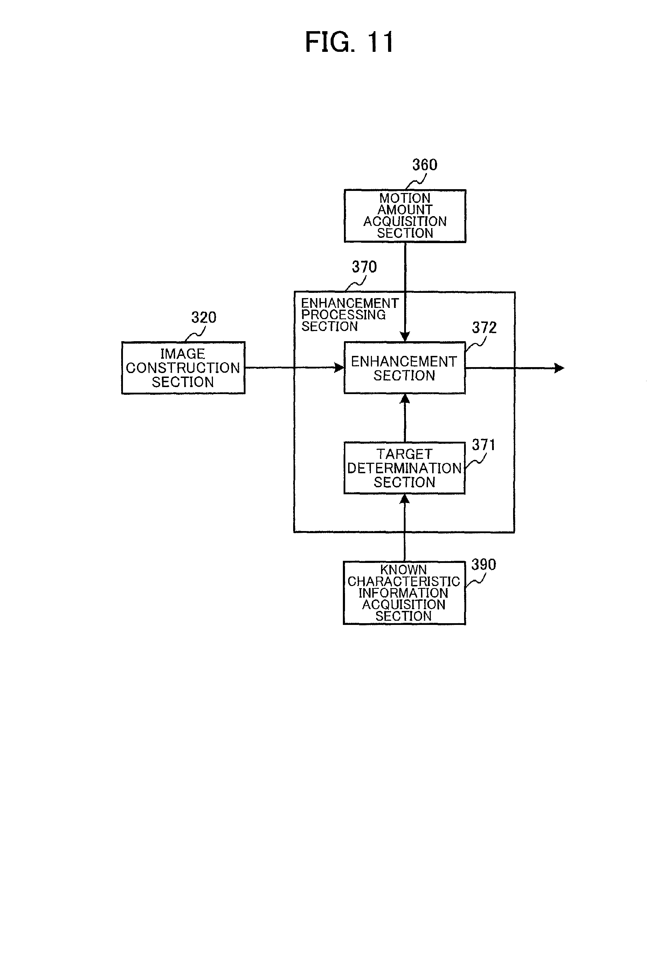

FIG. 11 illustrates a detailed configuration example of the enhancement processing section 370 according to the second embodiment. The enhancement processing section 370 includes a target determination section 371 and an enhancement section 372.

The known characteristic information acquisition section 390 acquires color information about the extraction target tissue (e.g., reddened part or discolored part) as the known characteristic information. The target determination section 371 determines an area that agrees with the color represented by the known characteristic information to be the target. For example, the target determination section 371 determines an area that has a high degree of redness as compared with a normal mucous membrane to be the reddened part. For example, the target determination section 371 determines an area for which the ratio "R/G" of the red (R) pixel value to the green (G) pixel value is larger than that of its peripheral area, to be the reddened part. In this case, the known characteristic information represents a condition whereby an area for which the ratio "R/G" is larger to a given extent (e.g., factor) than that of its peripheral area is determined to be the reddened part. Alternatively, the range of the ratio "R/G" or the hue value may be stored as the known characteristic information, and an area that falls under the range of the ratio "R/G" or the hue value may be determined to be the reddened part. An image frequency or structural information (e.g., size or shape) specific to the reddened part may also be used as the known characteristic information.

When the enhancement section 372 has determined that the motion amount is large, and screening observation is being performed, the enhancement section 372 enhances the color of the determined target. For example, the enhancement section 372 performs a process that increases the degree of redness (e.g., the ratio "R/G", or the chroma within the red hue range) of the reddened part, and decreases the degree of redness of the discolored part. When the enhancement section 372 has determined that the motion amount is small, and zoom observation is being performed, the enhancement section 372 performs a process that enhances the edge component of the image, or a process that enhances a specific frequency region through frequency analysis. The enhancement section 372 may perform a color enhancement process similar to that used during screening observation.

Although an example in which the target is determined has been described above, the enhancement process may be performed without determining the target. For example, a process that increases the degree of redness may be performed on an area having a high degree of redness when the motion amount is large, and the enhancement process that enhances a minute (microscopic) structure with a higher enhancement level may be performed when the motion amount is small. In this case, redness enhancement characteristics are stored as the known characteristic information, for example.

The detection target is not limited to the reddened part and the discolored part. For example, the detection target may be a polyp or the like. When the detection target is a polyp, a shape (or a color) specific to a polyp may be stored as the known characteristic information, and a polyp may be detected by performing a pattern matching process (or a color comparison process), for example. When the enhancement section 372 has determined that the motion amount is large, and screening observation is being performed, the enhancement section 372 may enhance a polyp by performing a contour enhancement process. When the enhancement section 372 has determined that the motion amount is small, and zoom observation is being performed, the enhancement section 372 may perform the color enhancement process in addition to the contour enhancement process.

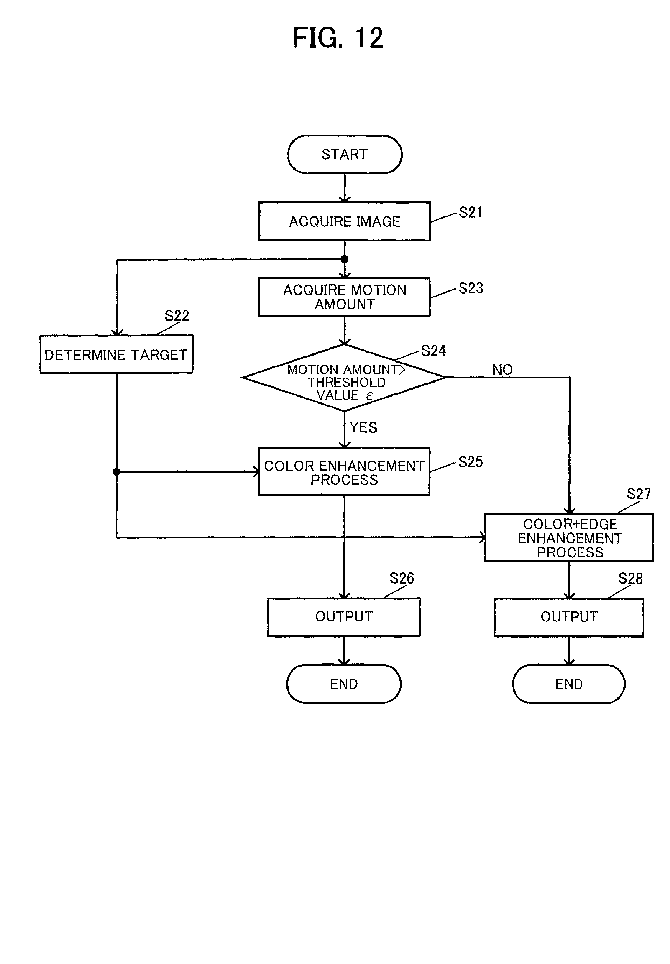

FIG. 12 is a flowchart when the process performed by the image processing section 301 is implemented by software. The captured image is acquired (step S1). The target is determined using the above method (step S22). The motion amount of the object is calculated from the captured image (step S23), and whether or not the motion amount (e.g., the magnitude of the motion vector) is larger than the threshold value $\varepsilon$ is determined (step S24). When the motion amount is larger than the threshold value $\varepsilon$ (i.e., when it has been determined that screening observation is being performed), the first enhancement process is performed on the captured image (step S25), and the resulting image is output (step S26). The first enhancement process enhances the color of the determined target. When the motion amount is equal to or smaller than the threshold value $\varepsilon$ (i.e., when it has been determined that zoom observation is being performed), the second enhancement process is performed on the captured image (step S27), and the resulting image is output (step S28). The second enhancement process enhances the color and the edge of the determined target.

According to the second embodiment, the known characteristic information acquisition section 390 acquires the known characteristic information that is information that represents known characteristics relating to the color of the object.

This makes it possible to perform the color enhancement process corresponding to the observation state based on the information about the color specific to the observation target. It is possible to perform the enhancement process so that a lesion or the like that differs in color from a normal area can be easily observed, by utilizing the information about the color specific to the observation target, for example.

The enhancement processing section 370 may perform the enhancement process on the object that agrees with the characteristics represented by the known characteristic information. For example, the target determination section 371 determines the target that agrees with the desired characteristics (e.g., color) represented by the known characteristic information from the captured image, and the enhancement processing section 370 performs the enhancement process on the determined target. Note that the target determination section 371 need not necessarily be provided as described above in connection with the first embodiment. The enhancement process may be performed based on the known characteristic information without determining the target.

More specifically, when the motion amount (e.g., the magnitude of the motion vector) is larger than the threshold value $\varepsilon$, the enhancement processing section 370 performs the enhancement process that enhances the color (e.g., the ratio "R/G", or the chroma within a given hue range) of the target. When the motion amount is smaller than the threshold value $\varepsilon$, the enhancement processing section 370 performs the enhancement process that enhances at least the structure of the target.

For example, the known characteristic information is information that represents known characteristics relating to the color of the reddened part for which the red component (e.g., ratio "R/G") is larger than that of a normal mucous membrane. When the motion amount is larger than the threshold value $\varepsilon$, the enhancement processing section 370 performs the enhancement process that enhances the red component (e.g., the ratio "R/G", or the chroma within the red hue range) of the reddened part that has been determined to be the target.

This makes it possible to determine the position of a lesion (e.g., reddened part or discolored part) having a specific color during screening observation in which the object (image) moves quickly. Since both a color and a structure can be enhanced during zoom observation in which the object (image) moves slowly, it is possible for the user to easily observe a minute lesion structure to which the user pays attention. It is thus possible to improve visibility in each observation state, and suppress a situation in which a lesion is missed while improving the diagnostic accuracy.

The embodiments to which the invention is applied and the modifications thereof have been described above. Note that the invention is not limited to the above embodiments and the modifications thereof. Various modifications and variations may be made of the above embodiments and the modifications thereof without departing from the scope of the invention. A plurality of elements described in connection with the above embodiments and the modifications thereof may be appropriately combined to implement various configurations. For example, some elements may be omitted from the elements described in connection with the above embodiments and the modifications thereof. Some of the elements described in connection with different embodiments or modifications thereof may be appropriately combined. Specifically, various modifications and applications are possible without materially departing from the novel teachings and advantages of the invention. Any term cited with a different term having a broader meaning or the same meaning at least once in the specification and the drawings can be replaced by the different term in any place in the specification and the drawings.

REFERENCE SIGNS LIST

2 to 4: fold, 10, 30: concave part, 20: convex part, 100: light source section, 101: white light source, 102: rotary color filter, 103: rotation driver section, 104: condenser lens, 200: imaging section, 201: light guide fiber, 203: illumination lens, 204, 205: objective lens, 206, 207: image sensor, 209: A/D conversion section, 210: memory, 212: connector, 250: lens driver section, 300: processor section, 301: image processing section, 302: control section, 310: image acquisition section, 320: image construction section, 323: luminance signal calculation section, 324: difference calculation section, 325: second derivative calculation section, 326: parameter calculation section, 327: storage section, 328: LUT storage section, 330: image storage section, 350: storage section, 360: motion amount acquisition section, 370: enhancement processing section, 371: target determination section, 372: enhancement section, 380: distance information acquisition section, 390: known characteristic information acquisition section, 400: display section, 500: external I/F section

**Claims**

1. An image processing device comprising:

    an image acquisition section that acquires a captured image that includes an image of an object;
    a motion amount acquisition section that acquires a motion amount of the object;
    a known characteristic information acquisition section that acquires known characteristic information, the known characteristic information being information that represents known characteristics relating to the object; and
    an enhancement processing section that performs an enhancement process that corresponds to the motion amount based on the known characteristic information.

2. The image processing device as defined in claim 1, further comprising:

    a distance information acquisition section that acquires distance information that represents a distance from an imaging section to the object when the imaging section captured the captured image,
    the enhancement processing section performing the enhancement process that corresponds to the motion amount based on the distance information and the known characteristic information.

3. The image processing device as defined in claim 2,
    the known characteristic information acquisition section acquiring the known characteristic information that is information that represents known characteristics relating to a structure of the object, and
    the enhancement processing section performing the enhancement process on a concave-convex part of the object that agrees with the characteristics represented by the known characteristic information.

4. The image processing device as defined in claim 3,
the enhancement processing section performing the enhancement process on the concave-convex part that has been determined to have a size larger than a first size threshold value when the motion amount is larger than a threshold value, and performing the enhancement process on the concave-convex part that has been determined to have a size smaller than a second size threshold value when the motion amount is smaller than the threshold value.

5. The image processing device as defined in claim 1,
the known characteristic information acquisition section acquiring the known characteristic information that is information that represents known characteristics relating to a color of the object.

6. The image processing device as defined in claim 5,
the enhancement processing section performing the enhancement process that corresponds to the motion amount on a target, the target being the object that agrees with the characteristics represented by the known characteristic information.

7. The image processing device as defined in claim 6,
the enhancement processing section performing the enhancement process that enhances a color of the target when the motion amount is larger than a threshold value, and performing the enhancement process that enhances at least a structure of the target when the motion amount is smaller than the threshold value.

8. The image processing device as defined in claim 6,
the known characteristic information being information that represents known characteristics relating to a color of a reddened part for which a red component is larger than that of a normal mucous membrane, and
the enhancement processing section performing the enhancement process that enhances the red component of the reddened part when the motion amount is larger than a threshold value.

9. The image processing device as defined in claim 1,
the enhancement processing section performing the enhancement process that corresponds to the motion amount on a target, the target being the object that agrees with the characteristics represented by the known characteristic information.

10. The image processing device as defined in claim 9,
the enhancement processing section performing a first enhancement process as the enhancement process on the object that agrees with the characteristics represented by the known characteristic information when the enhancement processing section has determined that the motion amount falls under a motion amount that corresponds to screening observation that is performed on the object, and performing a second enhancement process as the enhancement process on the object that agrees with the characteristics represented by the known characteristic information when the enhancement processing section has determined that the motion amount falls under a motion amount that corresponds to zoom observation that is performed on the object, the second enhancement process differing from the first enhancement process.

11. An endoscope apparatus comprising the image processing device as defined in claim 1.

12. An image processing method comprising:

acquiring a captured image that includes an image of an object;
acquiring a motion amount of the object;
acquiring known characteristic information, the known characteristic information being information that represents known characteristics relating to the object; and
performing an enhancement process that corresponds to the motion amount based on the known characteristic information.

13. An image processing program that causes a computer to perform steps of:

acquiring a captured image that includes an image of an object;
acquiring a motion amount of the object;
acquiring known characteristic information, the known characteristic information being information that represents known characteristics relating to the object; and

performing an enhancement process that corresponds to the motion amount based on the known characteristic information.

# FIG. 1

IMAGE PROCESSING DEVICE

360
MOTION AMOUNT
ACQUISITION
SECTION

310
IMAGE
ACQUISITION
SECTION

370
ENHANCEMENT
PROCESSING
SECTION

390
KNOWN
CHARACTERISTIC
INFORMATION
ACQUISITION
SECTION

# FIG. 2

# FIG. 3

EP 2 962 621 A1

EP 2 962 621 A1

# FIG. 4

# FIG. 5

# FIG. 6

## FIG. 7A

SURFACE 2
OF TISSUE

3 20 4

DISTANCE 10

30

## FIG. 7B

LOW-PASS FILTERING RESULTS

SUBTRACTION

## FIG. 7C

## FIG. 7D

CHANGE CHARACTERISTICS OF LOW-PASS FILTER
CORRESPONDING TO DISTANCE

RESPONSE        RESPONSE        RESPONSE

f                f                f

DISTANCE

(SHORT)                        (LONG)

## FIG. 8A

SURFACE 2
OF TISSUE

20
3
4

10
DISTANCE
30

## FIG. 8B

CLOSING PROCESS

## FIG. 8C

DETECTION OF CONCAVE PARTS

## FIG. 8D

OPENING PROCESS

## FIG. 8E

DETECTION OF CONVEX PARTS

## FIG. 8F

CHANGE RADIUS OF SPHERE
CORRESPONDING TO DISTANCE

O ⟶ O ⟶ o

⟶ DISTANCE
(SHORT)                    (LONG)

# FIG. 9

START

ACQUIRE IMAGE — S1

ACQUIRE DISTANCE
INFORMATION — S2

ACQUIRE MOTION
AMOUNT — S4

S3

DETERMINE TARGET

S5

MOTION AMOUNT>
THRESHOLD
VALUE $\varepsilon$

NO

YES

ENHANCEMENT
PROCESS (FIRST
ENHANCEMENT PROCESS) — S6

S8

ENHANCEMENT
PROCESS (SECOND
ENHANCEMENT PROCESS)

S7

OUTPUT

S9

OUTPUT

END

END

# FIG. 10

# FIG. 11

360

MOTION
AMOUNT
ACQUISITION
SECTION

370

ENHANCEMENT
PROCESSING
SECTION

320

IMAGE
CONSTRUCTION
SECTION

372

ENHANCEMENT
SECTION

371

TARGET
DETERMINATION
SECTION

390

KNOWN
CHARACTERISTIC
INFORMATION
ACQUISITION
SECTION

# FIG. 12

```
                    ┌─────────────┐
                    │    START    │
                    └──────┬──────┘
                           │
                    ┌──────▼──────────┐
                    │  ACQUIRE IMAGE  │────S21
                    └──────┬──────────┘
                           ●
                    ┌──────▼──────────┐
                    │ ACQUIRE MOTION  │────S23
                    │     AMOUNT      │
                    └──────┬──────────┘
        S22                │
  ┌─────────────────┐   ┌──▼─────────────┐ S24
  │ DETERMINE TARGET│   │ MOTION AMOUNT> │      NO
  │                 │   │   THRESHOLD    │────────────┐
  └─────────────────┘   │    VALUE ε     │            │
                        └──────┬─────────┘            │
                               │ YES                  │
        ●──────────────▶┌──────▼──────────┐ S25       │
                        │COLOR ENHANCEMENT│           │
                        │    PROCESS      │     ┌──────▼──────┐ S27
                        └──────┬──────────┘     │ COLOR+EDGE  │
                               │                │ ENHANCEMENT │
                        S26    │                │   PROCESS   │
                    ┌──────────▼──┐             └──────┬──────┘ S28
                    │   OUTPUT     │            ┌───────▼─────┐
                    └──────┬───────┘            │   OUTPUT    │
                           │                    └──────┬──────┘
                    ┌──────▼──────┐             ┌───────▼─────┐
                    │     END     │             │     END     │
                    └─────────────┘             └─────────────┘
```

27

**INTERNATIONAL SEARCH REPORT**

International application No.

PCT/JP2013/075627

A.  CLASSIFICATION OF SUBJECT MATTER
*A61B1/04*(2006.01)i, *A61B1/00*(2006.01)i, *G02B23/24*(2006.01)i

According to International Patent Classification (IPC) or to both national classification and IPC

B.  FIELDS SEARCHED

Minimum documentation searched (classification system followed by classification symbols)
A61B1/04, A61B1/00, G02B23/24

Documentation searched other than minimum documentation to the extent that such documents are included in the fields searched
Jitsuyo Shinan Koho        1922–1996   Jitsuyo Shinan Toroku Koho   1996–2013
Kokai Jitsuyo Shinan Koho  1971–2013   Toroku Jitsuyo Shinan Koho   1994–2013

Electronic data base consulted during the international search (name of data base and, where practicable, search terms used)

C.  DOCUMENTS CONSIDERED TO BE RELEVANT

| Category* | Citation of document, with indication, where appropriate, of the relevant passages | Relevant to claim No. |
|---|---|---|
| Y<br>A | JP 2012-100909 A  (Olympus Corp.),<br>31 May 2012 (31.05.2012),<br>paragraph [0037]<br>& US 2012/0120216 A1 | 1-3,5,6,<br>9-11,13<br>4,7,8 |
| Y<br>A | JP 2010-5095 A  (Fujinon Corp.),<br>14 January 2010 (14.01.2010),<br>paragraphs [0025], [0084] to [0087]<br>& US 2009/0322863 A1 | 1-3,5,6,<br>9-11,13<br>4,7,8 |
| Y | JP 2012-213552 A  (Fujifilm Corp.),<br>08 November 2012 (08.11.2012),<br>paragraphs [0005] to [0011]<br>(Family: none) | 3 |

☐  Further documents are listed in the continuation of Box C.       ☐  See patent family annex.

\*    Special categories of cited documents:
"A"  document defining the general state of the art which is not considered to be of particular relevance
"E"  earlier application or patent but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

Date of the actual completion of the international search
15 November, 2013 (15.11.13)

Date of mailing of the international search report
26 November, 2013 (26.11.13)

Name and mailing address of the ISA/
Japanese Patent Office

Authorized officer

Facsimile No.

Telephone No.

Form PCT/ISA/210 (second sheet) (July 2009)

28

## INTERNATIONAL SEARCH REPORT

| International application No. |
|---|
| PCT/JP2013/075627 |

**Box No. II      Observations where certain claims were found unsearchable (Continuation of item 2 of first sheet)**

This international search report has not been established in respect of certain claims under Article 17(2)(a) for the following reasons:

1. ☒ Claims Nos.: 12
   because they relate to subject matter not required to be searched by this Authority, namely:
   (See extra sheet)

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

**Box No. III      Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/075627

Continuation of Box No.II-1 of continuation of first sheet(2)

The invention in claim 12 is considered to be a method which is performed while an endoscope is inserted into a human body and maintained therein, and thus it "pertains to a method for treatment of the human body by surgery". Therefore, it relates to a subject matter on which the international search is not required to be carried out under the provisions of PCT Rule 39.1(iv).

Form PCT/ISA/210 (extra sheet) (July 2009)

**EP 2 962 621 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003088498 A

- JP 2005342234 A